# EUROPEAN PATENT APPLICATION

(11) **EP 1 943 999 A1**
(43) Date of publication of application: **16.07.2008**
(21) Application number: 08000407.0
(22) Date of filing: 10.01.2008
(51) Int. Cl.: A61H 23/02

(54) **Ultrasonic generator and skin care device using the same**

(30) Priority: 15.01.2007 JP 2007006244
(71) Applicant: MATSUSHITA ELECTRIC WORKS, LTD., Kadoma-shi, Osaka (JP)
(72) Inventor: Saida, Itaru, Kadoma-shi Osaka (JP); Matsusaka, Takeshi, Kadoma-shi Osaka (JP)
(74) Representative: Samson & Partner

(57) **Abstract**

An ultrasonic generator includes an ultrasonic vibrator (1) generating ultrasonic vibration, a horn (16) and a retainer mechanism attaching and fixing the horn in place. The horn includes a flat plate-like horn having opposite flat surfaces (16,16b), one of which forms a connection surface connected to the ultrasonic vibrator and the other of which forms a ultrasonic wave emitting surface from which ultrasonic waves are emitted toward a target point. A support portion for use in attaching and fixing the flat plate-like horn by means of the retainer mechanism (45) is formed on an outer circumferential surface (17) of the flat plate-like horn with a thickness smaller than that of the flat plate-like horn of the flat plate-like shape.

## Description

The present invention relates to an ultrasonic generator in which an ultrasonic vibrator is connected to a horn and a skin care device provided with the ultrasonic generator.

Conventionally, there is known an ultrasonic generator including a horn and an ultrasonic vibrator connected to the horn. The ultrasonic generator is configured to emit ultrasonic vibration to the outside through the horn by a mechanical vibration of the ultrasonic vibrator caused by an alternating electric field applied thereto.

The ultrasonic generator configured as above is adapted for use in, e.g., a skin care device that, when the horn is pressed against a skin surface, transmits ultrasonic vibration toward the skin surface or the inside of a living body to thereby provide a desired aesthetic effect (see, e.g., Japanese Patent Laid-open Application No. 2006-181292)).

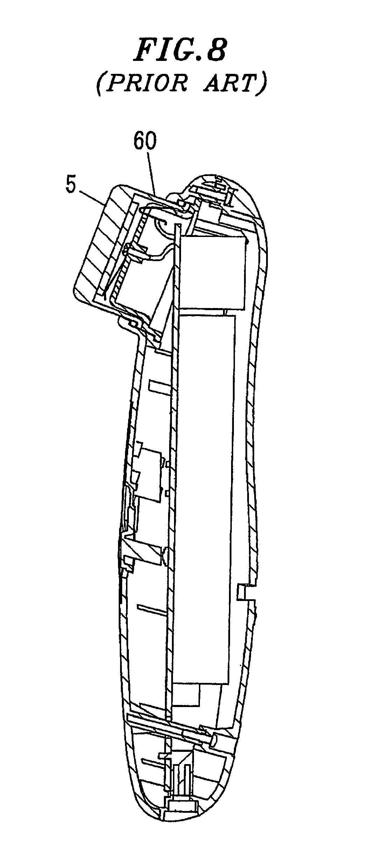

As shown in Fig. 8, in the conventional ultrasonic generator, a bottom-closed tubular horn having a side circumferential wall 60 is used as the horn 5 to which the ultrasonic vibrator 1 is connected. The bottom-closed tubular horn 5 provides an advantage in that the horn 5 can be stably attached and fixed at the side circumferential wall 60 thereof and another advantage in that the side circumferential wall 60 prevents liquid such as gel or the like used as an ultrasonic wave transmitting medium from infiltrating into the ultrasonic generator.

However, the ultrasonic generator provided with the bottom-closed tubular horn 5 suffers from the following problems. Specifically, the ultrasonic wave transmitting horn 5 is usually made of light metal, including aluminum, or light alloy. For this reason, it is difficult to produce a complex shape, i.e., a bottom-closed tubular shape, by machining in conformity with the strict dimensional requirements for assuring the reliable transmission of ultrasonic vibration.

Furthermore, an increased volume of metal is cut away in the process of producing the horn 5, which forms one cause of cost increase. Moreover, the horn 5 becomes largesized, consequently making the device bulky and heavyweight.

As a solution to these problems, it would be thinkable to form the horn 5 into a flat plate shape, in which case it becomes difficult to stably attach and fix the horn 5 in which an ultrasonic standing wave is generated.

In view of the problems noted above, the present invention provides an ultrasonic generator in which a horn of flat plate shape is used to make a device employing the same small-sized, lightweight and cost-effective and also to stably attach and fix the horn, and a skin care device using the ultrasonic generator.

In order to achieve the above object, the present invention provides an ultrasonic generator including: an ultrasonic vibrator generating ultrasonic vibration; a horn having a connection surface connected to the ultrasonic vibrator and an ultrasonic wave emitting surface from which ultrasonic waves are emitted toward a target point; and a retainer mechanism attaching and fixing the horn in place, wherein the horn comprises a flat plate-like horn having opposite flat surfaces, one of which forms the connection surface and the other of which forms the ultrasonic wave emitting surface, and wherein a support portion for use in attaching and fixing the flat plate-like horn by means of the retainer mechanism is formed on an outer circumferential surface of the flat plate-like horn with a thickness smaller that that of the flat plate-like horn.

With the ultrasonic generator noted above, the task of forming a metallic material into the flat plate-like horn becomes easy and the volume of metal cut away is reduced. This makes it possible to provide the ultrasonic generator in a cost-effective manner. Furthermore, use of the small-sized and lightweight flat plate-like horn is effective in reducing the size and weight of the device as a whole.

Moreover, owing to the fact that the support portion is formed on the outer circumferential surface of the flat plate-like horn, it is possible to stably attach and fix the flat plate-like horn through the support portion. It is also possible to avoid generation of stationary waves in the support portion, because the thickness of the support portion is smaller than that of the flat plate-like horn.

In the ultrasonic generator noted above, it is preferred that the support portion of the flat plate-like horn is formed into a flange shape at an end portion of the outer circumferential surface of the flat plate-like horn near the connection surface, the thickness of the support portion being 1/3 to 1/6 of one wavelength of the ultrasonic vibration propagated within the flat plate-like horn. This makes it possible to reliably prevent generation of stationary waves in the support portion and also to prevent the support portion from affecting the stationary waves generated in the flat plate-like horn.

Furthermore, owing to the fact that the support portion is formed at the end portion of the flat plate-like horn near the connection surface thereof with a thickness of 1/3 to 1/6 of one wavelength of the ultrasonic vibration, the end surface of the support portion opposite to the connection surface is positioned in alignment with or near the node of the stationary waves generated within the flat plate-like horn. Therefore, use of the support portion in fixing the flat plate-like horn does not affect the stationary waves generated within the flat plate-like horn which is attached and fixed in place through the flange-like support.

In the ultrasonic generator noted above, it is preferred that a plurality of rotation-proof cutout portions is formed on the connection surface of the flat plate-like horn. This prevents rotation of the flat plate-like horn when the ultrasonic vibration is propagated within the flat plate-like horn, thereby making it possible to stably attach and fix the horn.

In the ultrasonic generator noted above, it is preferred that the cutout portions are arranged at an equal interval along a circumference of a circle extending around a center point of the connection surface of the flat plate-like horn. The influence of the cutout portions on the propagation of ultrasonic vibration is suppressed by arranging the cutout portions at an equal interval. This is because the ultrasonic vibration is propagated from the center point C on the connection surface of the flat plate-like horn.

In the ultrasonic generator noted above, it is preferred that the retainer mechanism comprises a water-proof cover for attaching and fixing the flat plate-like horn through the support portion. This makes it possible for the water-proof cover to stably attach and fix the horn, while reliably preventing liquid from infiltrating into the device.

Furthermore, the present invention is directed to a skin care device comprising the ultrasonic generator configured as above, wherein the ultrasonic wave emitting surface of the flat plate-like horn forms a skin contact surface. This makes it possible to stably attach and fix the flat plate-like horn, thereby providing a small-sized, lightweight, cost-effective skin care device.

In accordance with the present invention, it is possible to reduce the size, weight and cost of a device by employing a flat plate-like horn. Moreover, the present invention is capable of providing an ultrasonic generator that can stably attach and fix the horn and a skin care device using the ultrasonic generator.

The objects and features of the present invention will become apparent from the following description of embodiment given in conjunction with the accompanying drawings, in which:
Fig. 1 is a side cross sectional view showing the whole view of a skin care device in accordance with one embodiment of the present invention;
Figs. 2A and 2B are front and rear views illustrating the whole view of the skin care device;
Fig. 3 is an exploded perspective view showing major parts of the skin care device;
Fig. 4 is a front view showing the major parts of the skin care device;
Fig. 5 is a side cross sectional view showing the major parts of the skin care device;
Figs. 6A and 6B are explanatory views illustrating the characterized parts of the skin care device;
Figs. 7A is a bottom view showing a horn of the skin care device and Figs. 7B and 7C are bottom views illustrating modified examples of the horn; and
Fig. 8 is a side cross sectional view showing a conventional skin care device.

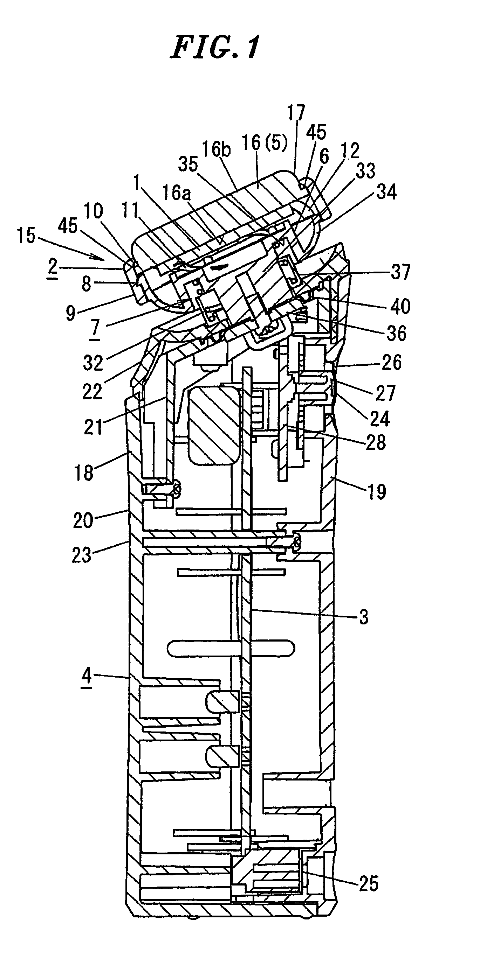

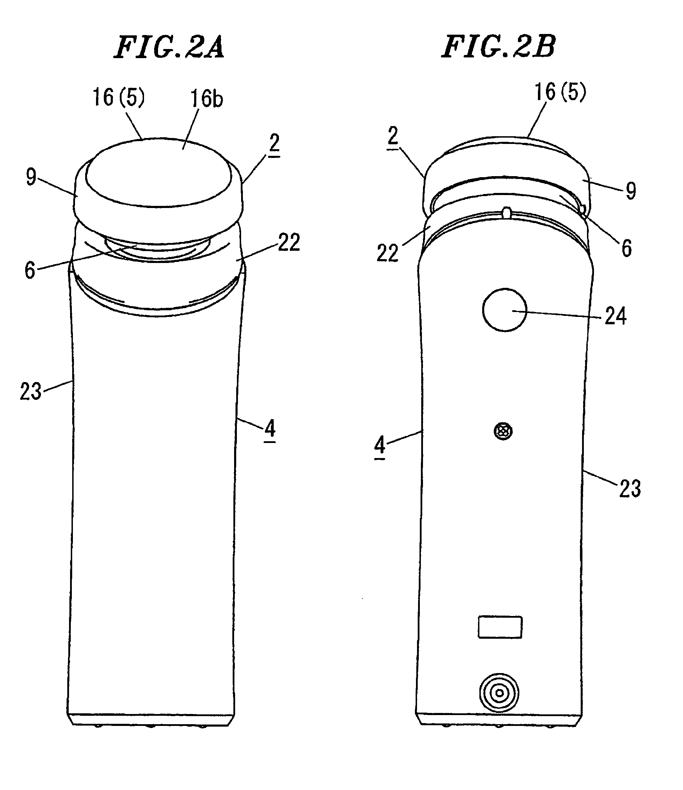

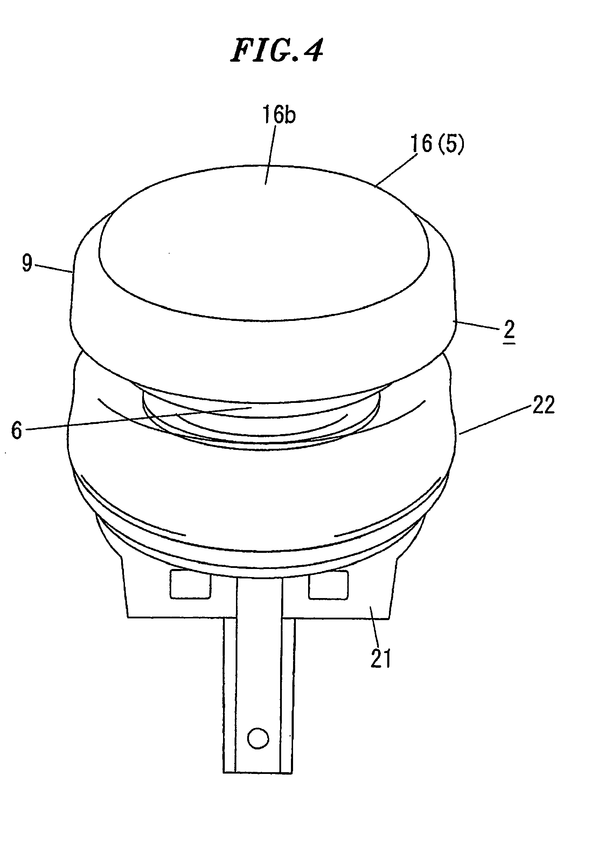

Embodiment of the present invention will now be described with reference to Figs. 1 to 8 which form a part hereof. Figs. 1, 2A and 2B show the whole view of a skin care device in accordance with an embodiment of the present invention. The skin care device in accordance with the present embodiment includes an ultrasonic generator and is designed to provide an aesthetic effect by transmitting the ultrasonic vibration emitted from an ultrasonic wave emitting surface 16b of the ultrasonic generator to a skin surface.

The skin care device in accordance with the present embodiment (i.e., the ultrasonic generator forming a part of the skin care device) includes, as its major components, a head block 2 with a built-in ultrasonic vibrator 1 and a grip block 4 having a built-in control circuit 3 that applies an alternating electric field to the ultrasonic vibrator 1 to generate ultrasonic vibration.

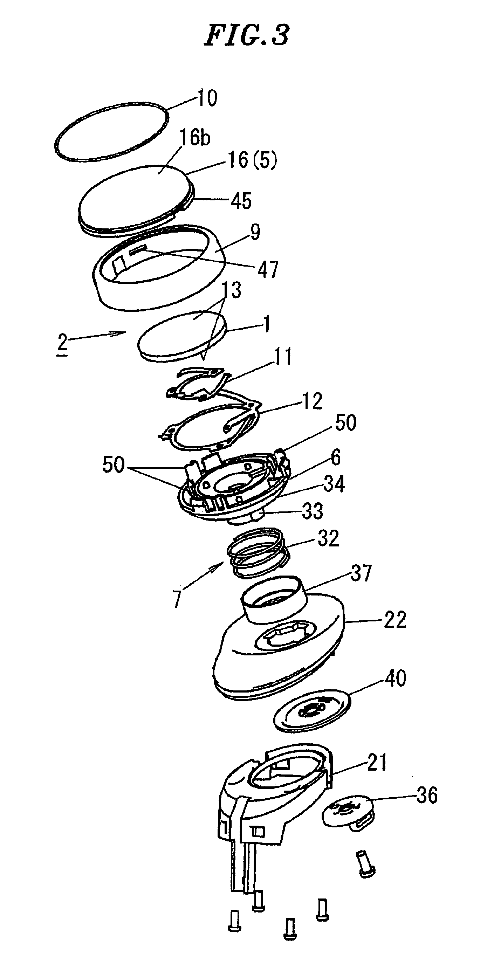

First, description will be made on the basic configuration of the head block 2. As illustrated in Figs. 3 to 5, the head block 2 includes a horn 5 of disc shape (hereinafter referred to as a "flat plate-like horn 16") made of metal such as aluminum or the like, a ultrasonic vibrator 1 adhesively fixed to one flat surface of the flat plate-like horn 16, a base portion 6 forming the bottom portion of the head block 2, and a water-proof cover 9.

The water-proof cover 9 engages with a flange-like support 45 projecting from the outer circumferential surface of the flat plate-like horn 16 and, at the same time, engages with a hook-like engagement part 8 protruding from the outer surface of the base portion 6. By attaching and fixing the flat plate-like horn 16 to the base portion 6 through the water-proof cover 9, the flat surface (hereinafter called an "ultrasonic wave emitting surface 16b") of the flat plate-like horn 16 opposite to the flat surface (hereinafter called a "connection surface 16a") to which the ultrasonic vibrator 1 is fixed can be exposed to the outside in a state that it protrudes obliquely and upwardly as viewed in the figure.

An O-ring 10 is interposed between the support portion of the flat plate-like horn 16 and the water-proof cover 9. The water-proof cover 9 and the O-ring 10 form a retainer mechanism 15 for attaching and fixing the flat plate-like horn 16 to the skin care device.

Also provided within the head block 2 are a vibrator-side connection terminal 11 and a horn-side connection terminal 12 for supplying electricity to the ultrasonic vibrator 1. The vibrator-side connection terminal 11 makes elastic contact with one of a pair of electrodes 13 (see Fig. 3) provided in the ultrasonic vibrator 1, so that the vibrator-side connection terminal 11 is directly and electrically connected to the corresponding electrode 13.

Furthermore, the horn-side connection terminal 12 makes elastic contact with the connection surface 16a of the flat plate-like horn 16, so that the horn-side connection terminal 12 is electrically connected to the other electrode 13 of the ultrasonic vibrator 1. The connection terminals 11 and 12 are both fixed to the base portion 6 and electrically connected to the control circuit 3 of the grip block 4 via a lead line (not shown).

Next, description will be made on the basic configuration of the grip block 4. The grip block 4 includes a bottom-closed tubular main housing 20 having a front housing 18 and a rear housing 19 screw-fixed to the front housing 18, a generally ring-shaped support base 21 screw-fixed to the opening of the main housing 20 and a top cover 22. The main housing 20, the support base 21 and the top cover 22 form an overall housing 23 of the grip block 4.

Received within the housing 23 are the control circuit 3, an operation switch 24 and a power source jack 25. The operation switch 24 includes a switch cover 26 attached to the rear housing portion 19, a switch boss 27 and a switch substrate 28. The switch substrate 28 and the power source jack 25 are electrically connected to the control circuit 3 via lead lines (not shown).

Next, description will be made on a support mechanism 7 for movably supporting the head block 2 on the grip block 4. In the present embodiment, the support mechanism 7 includes a coil spring 32 as an elastic member interposed in a compressed state between the base portion 6 forming the bottom portion of the head block 2 and the top cover 22 forming the top portion of the grip block 4. The bottom of the base portion 6 includes a columnar central penetration portion 33, a peripheral edge portion 34 surrounding the penetration portion 33, and an annular groove portion 35 formed between the penetration portion 33 and the peripheral edge portion 34.

The penetration portion 33 is inserted into the central aperture of the top cover 22. A disc-like stopper 36 is screw-fixed to the tip end of the penetration portion 33 within the top cover 22. The stopper 36 is a member that prevents slipping through the head block 2 by making contact with the inner surface of the top cover 22 through a water-proof cover 40 which will be described below.

A cylindrical water-proof member 37 is provided around the outer circumference of the coil spring 32 in such a manner as to surround the same. The coil spring 32 is pressed against the top cover 22 through the water-proof member 37. A sheet-like water-proof cover 40 is arranged inside the top cover 22 of the grip block 4. The water-proof member 37 and the water-proof cover 40 ensures that the liquid such as gel or the like used as an ultrasonic wave transmitting medium for skin care is prevented from infiltrating into the grip block 4, thereby avoiding circuit breakage or other troubles in a reliable manner.

Furthermore, the O-ring 10 arranged between the flat plate-like horn 16 and the water-proof cover 9 makes sure that the liquid such as gel or the like is prevented from infiltrating into the head block 2.

With the skin care device configured as above, if the operation switch 24 is turned on, an alternating electric field is applied to the ultrasonic vibrator 1 from the control circuit 3 through the connection terminals 11 and 12. The applied alternating electric field mechanically vibrates the ultrasonic vibrator 1 to thereby generate ultrasonic vibration. Then, when one touches skin of a body with the ultrasonic wave emitting surface 16b of the flat plate-like horn 16 while gripping the grip block 4 with one hand, the ultrasonic vibration generated in the ultrasonic vibrator 1 is transmitted to a skin surface or other destinations of a living body, thus attaining a desired aesthetic effect.

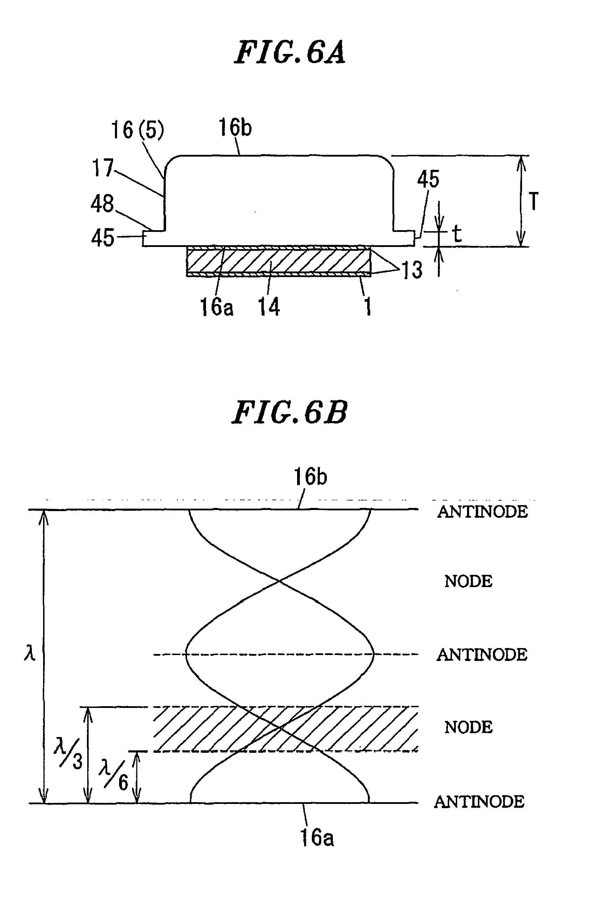

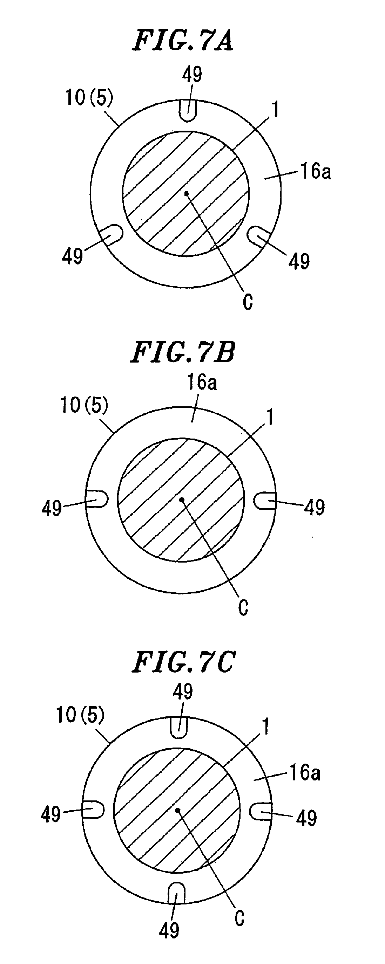

Next, description will be made on the characterized parts of the present invention with reference to Figs. 6 and 7. As set forth earlier, in the skin care device in accordance with the present embodiment, the flat plate-like horn 16 of disc shape having the oppositely positioned flat surfaces, i.e., the connection surface 16a and the ultrasonic wave emitting surface 16b, is used as the horn 5 for receiving the ultrasonic vibration from the adhesively fixed ultrasonic vibrator 1 and emitting the ultrasonic vibration toward a target point.

The ultrasonic vibrator 1 is fabricated by disposing a pair of electrodes 13 on the opposite flat surfaces of a disc-like piezoelectric material 14. One of the electrodes 13 of the ultrasonic vibrator 1 is adhesively fixed to the connection surface 16a of the flat plate-like horn 16 by use of an electrically conductive adhesive (not shown).

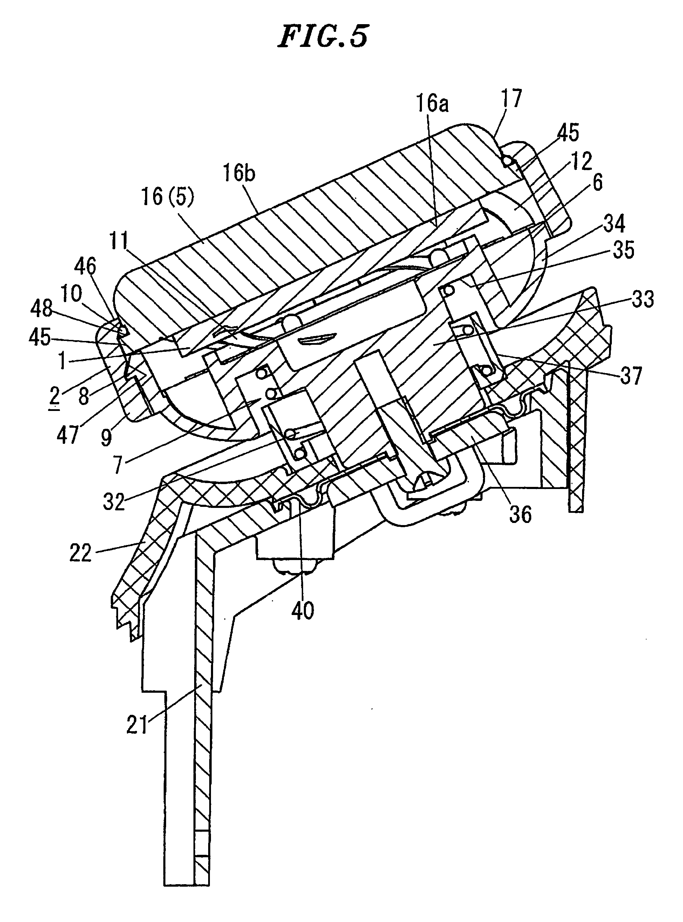

The flange-like support 45 is formed at the end portion of the outer circumferential surface 17 of the flat plate-like horn 16 near the connection surface 16a. A rib-like locking portion 46 protruding inwardly from the aperture circumference of one end of the water-proof cover 9 is locked to a surface 48 of the support portion 45 facing the ultrasonic wave emitting surface 16b, with the O-ring 10 interposed between the locking portion 46 and the end surface 48 (see Fig. 5).

A groove-like receiving portion 47 is formed on the inner circumferential surface of the water-proof cover 9. A hook-like engagement part 8 extending from the base portion 6 elastically engages at its lug portion with the groove-like receiving portion 47. At this time, the support portion 45 is sandwiched between the lug end of the engaging portion 8 and the locking portion 46 of the water-proof cover 9 through the O-ring 10, thereby positioning the flat plate-like horn 16 in place.

In this regard, the thickness t of the flange-like support 45 is made smaller than the thickness T of the flat plate-like horn 16 to avoid generation of stationary waves. Specifically, the thickness T of the flat plate-like horn 16 is set equal to the wavelength λ of the ultrasonic vibration propagated within the horn 16.

In contrast, the thickness t of the support portion 45 is set within the range of 1/3 to 1/6 of the thickness T of the flat plate-like horn 16 (i.e., 1/3 to 1/6 of the wavelength λ of the ultrasonic vibration). Therefore, as illustrated in Fig. 6B, stationary waves are generated within the flat plate-like horn 16 over the extent ranging from the connection surface 16a to the ultrasonic wave emitting surface 16b. The antinodes of the stationary waves exist at the connection surface 16a, the ultrasonic wave emitting surface 16b and the intermediate position between them (i.e., the position spaced 1/2 λ from the connection surface 16a toward the ultrasonic wave emitting surface 16b).

In contrast, the support portion 45 is formed so that the thickness t thereof measured from the connection surface 16a can fall within a range of 1/3 to 1/6λ. For this reason, no stationary wave is generated in the support portion 45. This makes it possible to stably attach and fix the flat plate-like horn 16 by use of the support portion 45.

The end surface 48 of the support portion 45 is positioned in a region encompassing a node of the stationary waves generated within the flat plate-like horn 16 (the hatched region in Fig. 6B). This region refers to a region in which the amplitude of the stationary waves is reduced to 50% or less of the greatest amplitude thereof. Therefore, presence of the support portion 45 has not great effect on the stationary waves of the flat plate-like horn 16. This allows the flat plate-like horn 16 to stably generate the ultrasonic vibration.

A plurality of rotation-proof cutout portions 49 is formed in the peripheral edge portion of the circular connection surface 16a of the flat plate-like horn 16. Rotation-proof protrusions 50 (see Fig. 3) extending from the base portion 6 are fitted into the cutout portions 49, which structure can reliably keep the flat plate-like horn 16 against rotation.

In the example illustrated in Fig. 7A, three cutout portions 49 are raidally arranged at an equal interval along the circumferential portion of the connection surface 16a of the flat plate-like horn 16. Alternatively, as shown in Figs. 7B and 7C, the cutout portions 49 may be two or four or more in number.

Although the ultrasonic vibration is propagated from the center point C of the connection surface 16a toward the peripheral portion, the influence of presence of the cutout portions 49 on the propagation of ultrasonic vibration is suppressed for the most part. This is because the cutout portions 49 are equally spaced from the center point C of the flat plate-like horn 16 and arranged at an equal interval along the circumferential portion of the flat plate-like horn 16.

The ultrasonic generator configured as above may be applied to a variety of devices other than the skin care device. Devices to which the present invention is applicable are not limited to the one for beauty care but may include the ones for performing various kinds of ultrasonic processing such as diagnosis, machining, bonding, cleansing, agent permeation acceleration, diffusion, dispersion, emulsification and atomization. The same effects as described above can be attained in this case.

While the invention has been shown and described with respect to the embodiment, it will be understood by those skilled in the art that various changes and modifications may be made without departing from the scope of the invention as defined in the following claims.

## Claims

1. An ultrasonic generator comprising:
an ultrasonic vibrator generating ultrasonic vibration;
a horn having a connection surface connected to the ultrasonic vibrator and an ultrasonic wave emitting surface from which ultrasonic waves are emitted toward a target point; and
a retainer mechanism attaching and fixing the horn in place,
wherein the horn includes a flat plate-like horn having opposite flat surfaces, one of which forms the connection surface and the other of which forms the ultrasonic wave emitting surface, and wherein a support portion for use in attaching and fixing the flat plate-like horn by means of the retainer mechanism is formed on an outer circumferential surface of the flat plate-like horn with a thickness smaller than that of the flat plate-like horn of the flat plate-like shape.

2. The ultrasonic generator of claim 1, wherein the support portion of the flat plate-like horn is formed into a flange shape at an end portion of the outer circumferential surface of the flat plate-like horn near the connection surface, the thickness of the support portion being 1/3 to 1/6 of one wavelength of the ultrasonic vibration propagated within the flat plate-like horn.

3. The ultrasonic generator of claim 1 or 2, wherein a plurality of rotation-proof cutout portions is formed on the connection surface of the flat plate-like horn.

4. The ultrasonic generator of claim 3, wherein the cutout portions are arranged at an equal interval along a circumference of a circle extending around a center point of the connection surface of the flat plate-like horn.

5. The ultrasonic generator of any one of claims 1 to 4, wherein the retainer mechanism includes a water-proof cover for attaching and fixing the flat plate-like horn through the support portion.

6. A skin care device comprising the ultrasonic generator of any one of claims 1 to 5, wherein the ultrasonic wave emitting surface of the flat plate-like horn forms a skin contact surface.
